# EUROPÄISCHE PATENTSCHRIFT

(11) **EP 4 157 171 B1**
(45) Veröffentlichungstag und Bekanntmachung des Hinweises auf die Patenterteilung: **04.03.2026**
(21) Anmeldenummer: 21729236.6
(22) Anmeldetag: 21.05.2021
(51) Int. Cl.: A61F 9/008, A61B 3/113

(54) **SYSTEM ZUR LASERBASIERTEN FEHLSICHTIGKEITSKORREKTUR UND VERFAHREN ZU DESSEN AUSRICHTUNG**
SYSTEM FOR LASER-BASED AMETROPIA CORRECTION, AND METHOD FOR THE ALIGNMENT THEREOF
SYSTÈME DE CORRECTION D'UNE AMÉTROPIE PAR LASER ET PROCÉDÉ POUR L'ORIENTER

(30) Priorität: 24.05.2020 DE 102020206423; 24.05.2020 DE 102020206426; 10.07.2020 DE 102020208676
(43) Veröffentlichungstag der Anmeldung: 05.04.2023
(73) Patentinhaber: Carl Zeiss Meditec AG, 07745 Jena (DE)
(72) Erfinder: VOGELSANG, Hartmut, 34399 Oberweser (DE); REINSTEIN, Dan Z., Greater London W1G 7LA London (GB); DEUTSCH, Christian, 99425 Weimar (DE); WUNDRICH, Ingo, 99423 Weimar (DE)
(74) Vertreter: Pearl Cohen EU
(86) Internationale Anmeldenummer: PCT/EP2021/063616
(87) Internationale Veröffentlichungsnummer: WO 2021/239606

(56) Entgegenhaltungen:
- WO-A1-2010/028663
- WO-A2-2009/033111
- US-A1- 2015 031 993

## Beschreibung

Die vorliegende Erfindung betrifft Laser Vision Correction (LVC) Systeme, also Systeme zur Fehlsichtigkeitskorrektur mittels Laserstrahlung, wobei der entsprechend enthaltene Behandlungs-Laser im Ultraviolettbereich emittiert, wie beispielsweise Excimer-Laser oder Festkörperlaser mit Wellenlängen zwischen ca. 193nm und 213nm, und/oder als Femtosekundenlaser oder Pikosedundenlaser ausgebildet ist. Diese Systeme sind typischerweise gepulste Systeme, also nicht kontinuierliche Wellen emittierende Systeme. Derartige LVC-Systeme werden eingesetzt, um mittels Photoablation die Cornea eines Patientenauges von deren Oberfläche ausgehend zu bearbeiten, bzw. unter einer weggeklappten Oberfläche der Cornea des Patientenauges ein Volumen ausgehend von der freigelegten Fläche aus zu bearbeiten. Alternativ können solche Syteme verwendet werden, um ein Lentikel in der Hornhaut zu separieren, sodass dieses anschließend entfernt werden kann. Die Erfindung betrifft weiterhin entsprechende Verfahren für die Ausrichtung bzw. Zentrierung solcher Systeme. In der vorliegenden Offenbarung werden LVC-System auch einfach als "Systeme" bezeichnet. Sofern nicht explizit angegeben, kann es sich bei einem System also um ein UV-Laserbasiertes oder ein Femtosekundenlaser/Pikosekundenlaser basiertes System handeln.

Herkömmliche LVC-Systeme, wie beispielsweise die MEL-Systeme der Carl Zeiss Meditec AG, die Amaris-Systeme der Schwind eye-tech solutions GmbH oder die Micron-Systeme der Excelsius Medical GmbH sind seit langem erfolgreich genutzte System zur Fehlsichtigkeitskorrektur. In der vorliegenden Offenbarung werden weitere Verbesserungen für derartige Systeme vorgeschlagen und erläutert.

In herkömmlichen LVC-Systemen ist typischerweise ein starres Laserstrahlführungssystem vorgesehen. Dies erleichtert zwar die sichere Laserstrahlführung, aber macht es nötig, den Patienten auf einer Patientenliege mittels dieser Patientenliege unterhalb einer festen Systemapertur in x,y,z Koordinate zu verschieben, bis das zur Behandlung vorgesehene Patientenauge korrekt zur optischen Achse des Systems positioniert ist. Eine Ausnahme bildet das in US 2013/0226157 A1 beschriebene System, in dem der an sich starre Laserarm als Ganzes über dem Patienten positioniert wird, jedoch in einer Art und Weise, dass nach wie vor die Positionierung des Patienten über die Patientenliege erforderlich ist. Letzteres erfordert aus Sicherheitsgründen häufig, dass die Patientenliegen elektrisch und/oder mechanisch mit der Lasergrundeinheit verbunden sind, was wiederum eine Systemzulassung erzwingt und einen großen Stellplatz erfordert.

Auch erfolgt in herkömmlichen LVC-Systemen üblicherweise eine manuelle, statische Ausrichtung des Auges bzgl. Zyklorotation, d.h. ohne automatische Korrektur mit Hilfe von Registrierdaten, durch Drehen des Patientenkopfes auf der Liege unter Sichtkontrolle. Dies begrenzt die erzielbare Genauigkeit und kann dann zu unerwünschten Abweichungen führen, wenn eine Drehung des Kopfes des Patienten während der Behandlung erfolgt, ohne dass eine Korrektur der dadurch verursachten Zyklorotation erfolgt.

In manchen herkömmlichen LVC-Systemen sind zudem Kontaktinterfaces zur Fixierung des Auges bekannt. Sie werden dabei in solchen LVC-Systemen, wie beispielsweise in US 2013/0226157 A1 und US 9592156 B2 beschrieben, lediglich zur Stabilisierung des Auges implementiert, und übernehmen keine aktive Rolle. In vielen Systemen wird hingegen ganz ohne Kontaktinterfaces gearbeitet.

Mit der Einführung von spot-scanning LVC-Systemen wurden große Arbeitsabstände zwischen Laseraustritts-Apertur und Auge realisiert. Dies geschah auch vor dem Hintergrund der Verwendung von Mikrokeratomen, die mit dem Patienten auf dem Patientenbett des Systems verwendet wurden, um den LASIK Flap, also eine wegklappbare Öffnung der Cornea, zu schneiden. U.a. auch dadurch wurden große Arbeitsabstände gefordert. Später wurden Eye-Tracker zur Registrierung und Kompensation der Augenbewegungen eingeführt, und damit die Augenbewegungen während der Ablation kompensiert, da das Auge nicht fixiert ist. Auch das damit einhergehende optische System-Gesamtkonzept - welches bei den verschiedenen Systemen im Grunde sehr ähnlich ist - kann als ungünstig betrachtet werden.

In der Anwendung ergeben sich für das Eye-Tracking verschiedene technische Herausforderungen. Insgesamt ist die Registrierungsgeschwindigkeit der Augenbewegung limitiert und die Einstellung der Scanner-Spiegel zur Korrektur der Pulskoordinate zeitlich endlich. Im Zusammenhang mit der Systemperformance ist die Reaktion auf die Augenbewegung ("response time") in der Regel verzögert und damit in ihrer Genauigkeit begrenzt. Bei herkömmlichen, schnellen Eye-Tracker Systemen (ca. 1000 Hz Repetitionsrate) ist dies ausreichend für die lateral-Korrektur (x,y Verschiebung; "1. und 2. Eye-Tracking Dimension"). Allerdings können auch die schnellsten herkömmlichen Systeme zu Limitierungen führen, insbesondere wenn die Eye-Tracker Geschwindigkeit unter dem Limit für die Abtastfrequenz liegt. In manchen Systemen wird basierend auf den vorhergehenden Bewegungs-Trajektorien des Auges eine Prognose darüber gemacht, wohin das Auge sich bewegen wird ("7. Eye-Tracking Dimension"). Dies ist im Rahmen einer Näherung möglich, da Augen- Sakkaden/Nystagmen im weitesten Sinne statistischen Bewegungen des Auges entsprechen. Dies zeigt auch, dass mit herkömmlichen Technologie eine Erhöhung der Repetitionsrate mit technischen Herausforderungen verbunden ist, obwohl diese für bestimmte Applikationen und in bestimmten Ablationszeitfenstern interessant wäre (thermal controlled/mild ablation).

Des Weiteren kann ein rein laterales Tracking eine Limitierung darstellen, da die Rotation des Auges um die z-Achse ("dynamische Zyklorotation"; 6. Eye-Tracking Dimension") und die Rollbewegungen um die horizontale und vertikale Augenachse (3. und 4. Eye-Tracking Dimension") zur Erreichung einer bestmöglichen Genauigkeit zu berücksichtigen sind. Zudem kann auch der Abstand (z-Distanz) zur Laser-Austrittsapertur variieren, was durch entsprechendes Tracking ebenfalls kompensiert werden kann (5. Eye-Tracker Dimension).

Trotz aller technischer Feinheiten und Korrekturmöglichkeiten sind die herkömmlichen Systeme in manchen Fällen darin limitiert, exakt auf eine Änderung der Augenposition zu reagieren: Die limitierte Güte der Registrierung und die Geschwindigkeit der Registrierung und Korrektur können dabei einen maßgeblichen Einfluss haben. Der Einfluss auf die refraktiven Ergebnisse ist jedoch im Normalfall sehr gering und kaum nachweisbar.

Im Zusammenhang mit dem Eye-Tracking können sich aber bei unkooperativen Patienten, bei denen Fixationsinstabilität, Nervosität, kognitive Defizite oder Wahrnehmungsprobleme des Fixationstargets bestehen, weitere Herausforderungen ergeben. In manchen Fällen kann es für die Augenchirurgen geboten erscheinen, die Augen des Patienten während der Ablation manuell mittels einer Klammer und/oder eines Schaumspatels zu fixieren, um eine präzise Ablation zu ermöglichen. Dies kann dazu dienen, eine Augenbewegung außerhalb der sog. (limitierten) "Eye-Tracker Hotzone" zu vermeiden, da ansonsten ein Anhalten des Systems erforderlich sein kann.

Auch ist bei LVC-Systemen stets sicherzustellen, dass die Erzeugung prismatischer Fehler durch den Eye-Tracker zu nennen zu vermeiden ist. Wird dies nicht sichergestellt, kann der unerwünschte Fall eintreten, dass die Ablationsprofile nicht in der korrekten Ebene, d.h. nicht auf die Oberflächennormale, also senkrecht zur visuellen Achse, appliziert werden. Dies kann dadurch begünstigt werden, dass der Patient bevorzugt in eine weitgehend feste, aber "falsche" Richtung fixiert, also z.B. permanent in eine feste Richtung schaut, die nicht dem Zentrum der "Fixationswolke" entspricht (während der Operation kann der Patient das Fixationstarget je nach Refraktionsdefizit und Behandlungsdauer nicht mehr scharf sehen).

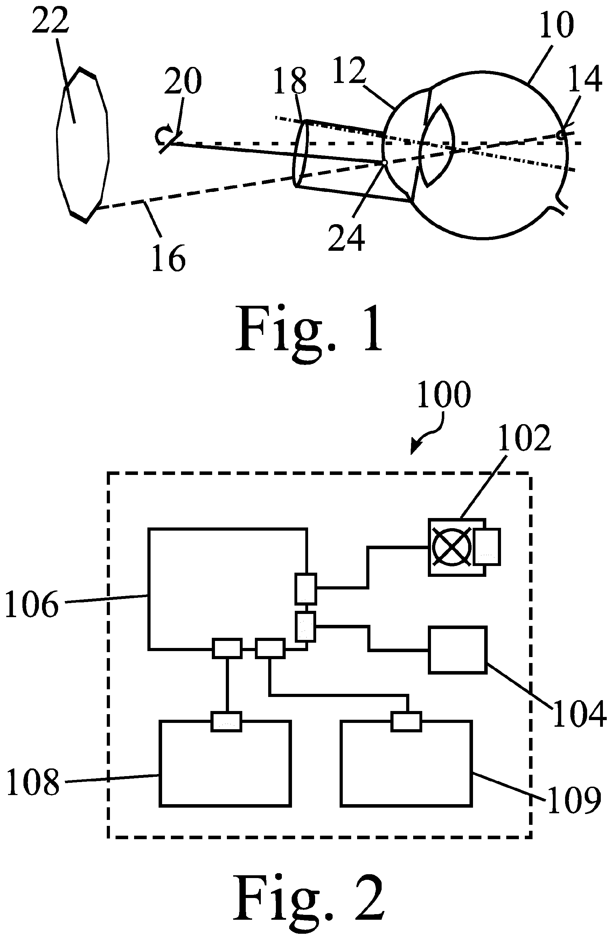

Figur 1 zeigt in einer schematischen Darstellung das Prinzip der Entstehung prismatischer Korrekturfehler (Tip/Tilt) durch den unzureichend genauen Einsatz eines Eye-Trackers, wenn der Patient nicht auf das Zentrum der "Fixationswolke "fixiert. Dabei ist in Figur 1 ein Patientenauge 10 mit Cornea 12 und Fovea 14 dargestellt, sowie die optische Achse 16 des Patientenauges bzw. die visuelle Achse 16, ein Ablationsprofil 18, ein Scansystem 20 und ein Fixierelement 22, auf welches der Patient den Blick zu fixieren hat.

Fixiert der Patient das Auge 10 nicht auf das Zentrum sondern beispielsweise auf einen Randbereich des Fixierelements 22, kann dies zur Folge haben, dass das Ablationsprofil 18 nicht korrekt entlang der notwendigen Behandlungsachse appliziert (z.B. visuelle Achse 16; definiert durch den ophthalmischen Pol (ophthalmic pole, OP) und Fixation des Patienten) und damit nicht normal zur visuellen Achse. Die Verhältnisse sind in Figur 1 stark übertrieben dargestellt.

Auch funktionieren Eye-Tracker nicht bei allen Augen gleichermaßen zuverlässig, da manche Augenfarben einen unzureichenden Kontrast aufweisen können. Sofern die Verwendung des Eye-Trackers nicht möglich ist, können Augenchirurgen dazu angehalten sein, die Operation abzubrechen oder ohne Unterstützung des Eye-Trackers fortzufahren, was jedoch höhere Anforderungen an die Fähigkeiten des Chirurgen stellt.

Bei herkömmlichen Systemen können oftmals nicht, oder nur sehr bedingt, konstante Umgebungsbedingungen über dem Operations-Situs eingestellt werden. Es ist jedoch bekannt, dass der Einfluss variierender Umgebungsbedingungen, wie Luftfeuchtigkeit und damit einhergehende Änderungen im Hydrierungszustand der Cornea, die Zusammensetzung der Luft (bei Ausdünstungen z.B. von Lösungsmitteln) oder die Temperatur erheblich sein können in Bezug auf die refraktiven Ergebnisse. Es ist auch bekannt, dass es von Vorteil ist, für die Erhaltung des Hydratationszustands der Hornhaut während der Ablation zu sorgen, bzw. deren Austrocknung zu vermeiden (siehe z.B. R. R. Krueger, M. Campos, X. W. Wang, M. Lee, P. J. McDonnell, "Corneal Surface Morphology Following Excimer Laser Ablation With Humidified Gases" , Arch Ophthalmol. 111, 1993). Für die Hydrierung sind typischerweise zwei Effekte zu unterscheiden: a) die physiologischen Unterschiede der Hydrierung der Hornhaut als Streuung zwischen verschiedenen Patienten, und b) die Erhaltung der Hydratation während der Ablation selber. Beide Einflüsse führen zu einer erhöhten Streuung des refraktiven Ergebnisses (über beispielsweise eine erhöhte Streuung in der Vorhersage "attempted vs. achieved"). In der Fachliteratur gibt es hier vielfältige Untersuchungen. Insbesondere der Einfluss der Hydration der Hornhaut ist demnach signifikant.

Ein weiterer Einflussfaktor auf die refraktiven Ergebnisse hängt mit der Menge und Ansammlung von Ablationsprodukten ("Debris") über der ablatierten Hornhaut, also dem Operations-Situs, zusammen. Es ist hinlänglich bekannt, dass es zur Absorption und Streuung der eingestrahlten UV Ablationspulse im Debris kommen kann. Dadurch kann die effektive Pulse-Fluence, die den Ablationsprozess maßgeblich steuert, in unkontrollierter Art und Weise modifiziert werden. Dies kann zu erheblichen Fluence-Abweichungen in der Abfolge der Ablationspulse führen. Bei Myopie-Behandlungen kann dies zur unerwünschten Entstehung zentraler Aufsteilungen der Hornhaut post-operativ (sogenannten central islands) führen. Herkömmliche Systeme verfügen daher meistens über eine Absaugung oder kombinierte Luftzuführung und Absaugung für das Debris. Ein großer Abstand der Zu- bzw. Abführungen kann jedoch einer effektiven Entfernung des Debris entgegenstehen. Im Prinzip wäre hierzu ein Austausch des gesamten Luftvolumens über der behandelten Hornhaut zwischen aufeinanderfolgenden Pulsen bei 500 Hz bis 1000 Hz Pulsrepetitionsrate vorteilhaft. Ansonsten kann es - bei nicht-optimaler oder gerichteter Absaugung der Ablationsprodukte - zu "schiefen" Ablationen und somit z.B. induziertem Koma oder SIA (surgically induced astigmatism) kommen, was es zu vermeiden gilt.

Bei Systemen, die zusätzlich Luft zuführen, gilt es darüber hinaus eine Dehydrierung der Hornhaut zu vermeiden. Dies kann in der Regel nur bedingt vermieden werden. Insgesamt kann der Operations-Situs durch die offene Anordnung in herkömmlichen Systemen auch nicht ohne weiteres vom restlichen operativen Umfeld entkoppelt werden (z.B. Raumluftströmungen).

Die sterile und sichere Ablage des Flaps ist von herausragender Wichtigkeit für eine LASIK Prozedur. Ein Flap ist typischerweise nur 100µm dick und nach dem LASIK Schnitt nur durch ein sehr schmales "Scharnier", dem Hinge, an der Hornhaut befestigt. Die Erhaltung der Hydrierung des Flaps ist sehr wichtig aus pathologischen Gründen aber auch zum Erhalt der Form des Flaps, da dehydrierte Flaps innerhalb von Sekunden schrumpfen können. Ein geschrumpfter Flap kann unter Umständen nach der Behandlung an Passgenauigkeit hinsichtlich des stromalen Betts einbüßen (was auch der Formänderung der Stroma-Oberfläche durch die Ablation geschuldet sein kann), was bei mangelhafter Berücksichtigung zu post-operativen Komplikationen führen kann (z.B. "epithelial ingrowth"). Flaps sollten auch möglichst nicht gefaltet, gezogen oder anderweitig gestresst werden. Die früher verwendete "Calzone-Technik" ist daher bei Experten heute kaum noch in Verwendung. Außerdem sollte auch vermieden werden, dass der Flap in möglicherweise nicht-sterilen Bereichen des Auges zu liegen kommt. Dies kann trotz der sterilen Vorbereitung des Auges z.B. durch Tränenfilm oder Kontakt zu nicht sterilen Teilen der Lider geschehen.

In Ermangelung einer in die herkömmlichen Systeme integrierten Lösung schneiden sich Anwender teilweise selber Flap-Ablagen aus sterilen Schaumspateln (oder ähnlichem Material), die dann befeuchtet werden und als sichere und sterile Ablage für den empfindlichen Flap dienen. Es wird also eine Lösung gesucht, um dies zuverbessern.

Aufgrund der relativ großen Arbeitsabstände Δ von herkömmlichen LVC-Systemen gibt es dort kaum einen Unterschied in der Fokussierungsebene. Diese ULV-LVC-Systeme können daher als nahezu bildseitig telezentrisch angesehen werden. Bei herkömmlichen LVC-Systemen, welche typischerweise einen Arbeitsabstand (Abstand der Geräteaustritts-Apertur-Kontur zum Auge) von etwa 250 mm aufweisen, treffen die Strahlen unter einem erheblichen Winkel auf die Hornhaut auf, da der typische Krümmungsradius RC des menschlichen Auges bei etwa 7.86 mm liegt. Dies hat den weiteren Nachteil, dass auch der optische Akzeptanzwinkel für die Rückführung von Reflexionen an der Hornhaut in das optische System sehr gering ist, was zu erheblichen Einschränkungen heutiger Systeme führt und eine Nutzung der Reflexionen an der Hornhaut in der Regel ausschließt oder auf ein minimales Maß reduziert.

Es werden nun weitere Nachteile von herkömmlichen LVC-Systemen beschrieben, die sich weitgehend direkt aus den optischen Konzepten und der daraus resultierenden Geometrie der Ablation ergeben.

Durch einen schrägen Einfall der Laserstrahlung auf die Hornhaut kommt es bei herkömmlichen Systemen zu Verlusten in der Fluence, also der Energiedichte des Laserpulses, die für die Ablation maßgeblich ist. Hier sind zwei Effekte zu berücksichtigen: Die Verluste aus Abweichungen des Pulse Ablations-Abdrucks ("ablation footprint) durch die lokale Geometrie der Hornhaut am Ort des Ablations-Pulses ("Geometriefaktor") und die Fresnel-Verluste bei Einstrahlung von Licht an Grenzflächen mit unterschiedlichem Brechungsindex (Luft, Hornhaut), die durch die Fresnel-Gleichungen berechnet werden können. Diese Effekte sind hinlänglich im Stand der Technik bekannt.

Die Pulse Ablations-Form ("Pulse ablation shape"; entspricht ablationswirksamer Fluenceverteilung des eingestrahlten Ablationslaser-Pulses auf einer Ebene senkrecht zur Einfallsrichtung) wird durch die Geometrie der Einstrahlung auf der Hornhaut verformt zum "Pulse ablation footprint on cornea" und damit ändert sich die Fluence-Verteilung gegenüber der eingestrahlten "Pulse ablation shape".

Die Fresnel-Verluste können mit Hilfe der Fresnel-Gleichungen unter Kenntnis der Brechungsindizes von Luft und Hornhaut (bzw. Stroma) und der Einfallswinkel berechnet werden, wobei die Polarisation der Laserstrahlung zu berücksichtigen ist.

Aufgrund des großen Arbeitsabstands herkömmlicher LVC-Systeme zum Patientenauge und dem damit einhergehenden kleinen Akzeptanzwinkel der Fokussierungsoptik ist es nur sehr begrenzt möglich oder gar ganz unmöglich, Rückreflexe von der Hornhaut des Patientenauges zur Analyse und insbesondere zur Zentrierung zu nutzen. Der Einfluss ungenauer Zentrierung ist bekannt und bereits vielfach in der Literatur diskutiert. Die häufig vertretene Meinung, dass Zentrierungsfehler, also Abweichungen des Ablationszentrums von den Sollpositionen auf der Hornhaut, wie typischerweise durch den "Ophthalmic Pole" für Zentrierung auf der visuellen Achse, die im Folgenden Dezentrierungen genannt werden, keinen Einfluss auf sphärische Korrekturen hätten, ist physikalisch nur in bestimmten Fällen, wie sphärische Korrekturen auf sphärischen Hornhäuten, zutreffend. Dabei wird aber u.a. die Sehphysiologie nicht berücksichtigt. Dezentrierungen können i.d.R. zu einer Verschiebung der physiologischen Sehachse führen. Das Auge wird bei der Verarbeitung des Seheindrucks im Gehirn durch die Augenmuskeln so "gedreht", dass das Licht weiterhin in den Punkt des schärfsten Sehens fällt, was im Grunde den prismatischen Versatz kompensiert ("tip/tilt"). Dies kann z.B. beim binokularen Sehen (Stereopsis) zu Problemen führen, die z.B. aus Untersuchungen zu unzureichend zentrierten Brillengläsern bekannt sind. Insbesondere bei asphärischen Korrekturen auf ellipsotorischen Hornhäuten, was dem realen, tatsächlichen Szenario entspricht, kann eine Dezentrierung auch rein physikalisch zu einem Nichterreichen der angestrebten Korrektur führen. Dezentrierungen können demnach einen erheblichen Einfluss auf die Ergebnisse von "Customized Ablation" haben, da es dadurch zur Induktion höherer Aberrationen ("Nachtsehprobleme", etc.) und damit auch zum Einfluss auf das refraktive Ergebnis kommt. Eine möglichst exakte Zentrierung ist somit für ein gutes Ergebnis bei Topographie- als auch bei Wellenfront-Korrekturen von zentraler Bedeutung.

Aberrationen (oder optische Moden) koppeln unter Dezentrierung. Durch die Kopplung von Sphäre und Zylinder an Aberrationen höherer Ordnung (Coma, Sphärische Aberration, Astigmatismen höherer Ordnung), auch jene, die in natürlichen (asphärischen) Augen vorkommen, sind Dezentrierungen in realen Augen, aber auch bei rein sphäro-zylindrischen Korrekturen, oftmals kritisch. Z.B. koppelt Coma bei Dezentrierung an Astigmatismus und Defokus oder sphärische Aberration an Coma, Astigmatismus und Defokus. Es sollen hier einige Beispiele gegeben werden, die zunächst nur die Effekte für die primären Aberrationen (bis 4. Ordnung) aufzeigen. Die Berechnungen erfolgen aus der Koordinatentransformation von optischen Moden:
- eine Verschiebung von 0.25 µm mit Coma Z(3,1) um 0.3 mm führt zu ca. -1/8 D Defokus
- eine Verschiebung von 0.25 µm mit Coma Z(3,1) um 0.3 mm (horizontal/vertikal) führt zu 1/8 D Kardinal Astigmatismus (Z(2,2) / Z(2,-2))
- eine Verschiebung von 0.5 µm mit Coma Z(3,1) um 0.5 mm führt zu ca. -0.3 D Defokus
- eine Verschiebung von 0.4 µm mit Coma Z(3,1) um 0.5 mm (horizontal/vertikal) führt zu 0.3 D Kardinal Astigmatismus (Z(2,2) / Z(2,-2))
- eine Verschiebung von 0.6 µm mit Sphärischer Aberration Z(4,0) um 0.4 mm führt zu ca. -1/8 D Defokus
- eine Verschiebung von 0.6 µm mit Sphärischer Aberration Z(4,0) um 0.4 mm (horizontal/vertikal) führt zu ca. 1/8 D Kardinal Astigmatismus (Z(2,2) / Z(2,-2))

Bisher wurden nur Betrachtungen für optische Moden gemacht, welche sich in Ablationsprofilen in der optischen Zone manifestieren. Die Übergangszonen wurden noch nicht erwähnt. Dezentrierungen bedeuten in diesem Zusammenhang aber auch, dass Übergangszonen in die optisch aktive Zone hineinreichen können, insbesondere bei Hyperopie-Korrekturen. Dies kann sodann zu Störungen führen (bekannt als "night vision complaints post surgery", hier ist nicht Nachtmyopie gemeint), insbesondere bei mesopischen bis skotopischen Lichtbedingungen, und entsprechend mit einer Patientenunzufriedenheit einhergehen.

Pupillenzentrierungen (Zentrierung zum CSC, "Corneal Sighting Center") lassen sich in der refraktiven Chirurgie mittels Augenverfolgungssystemen ("Eye-Tracker") als integrierter Pupillenerkennung gut und sicher bewerkstelligen.

Allerdings ist diese Art der Zentrierung nicht die bevorzugte Wahl, da es in der Fachwelt mittlerweile unstrittig ist, dass eine Zentrierung zum Ophthalmic Pole (visuelle Achse, Coaxially Sighted Corneal Light Reflex, "CSCLR", Bedingung, s.u.) bzw. zum Vertex korrekt wäre und einer Pupillenzentrierung vorzuziehen wäre. Kleine und mittlere Myopie-Korrekturen sind hier erfahrungsgemäß sehr unkritisch. Schwieriger wird es bei größeren Astigmatismen und Myopie-Korrekturen und insbesondere Hyperopie-Korrekturen. Hyperope Augen sind nämlich typischerweise durch einen nicht zu vernachlässigbaren Winkel zwischen Pupillarachse und visueller Achse des Auges ("angle kappa") charakterisiert. Corneal Sighting Center und Ophthalmic Pole liegen hier nicht mehr ausreichend nah beieinander, was zu einem Unterschied zwischen einem Winkel Lamda"angle lambda" und einem Winkel Kappa "angle kappa" führt. In der Regel bezeichnet der Winkel Lambda den Winkel zwischen der Pupillenachse oder Pupillarachse, (engl.: pupillary axis) und der "line of sight" sowie der Winkel Kappa "angle kappa" den Winkel zwischen der Pupillenachse und der "visual axis". Vgl. dazu "Handbook of Visual Optics Vol. 1", CRC Press Taylor & Francis Group, Ed. Pablo Artal, Vol 1, Chapter 17 (by D. A. Atchison), or "Investigation of the isoplanatic patch and wavefront aberration along the pupillary axis compared to the line of sight in the eye", M. Nowakowski, M. Sheehan, D. Neal, A. V. Goncharov, Biomedical Optics Express, Vol. 3, 2.

Außerdem ist die Pupille und das Pupillenzentrum kein fester Punkt, der zweifelsfrei mit einer Markierung versehen werden könnte. Sowohl die Pupille als auch das Pupillenzentrum variieren regelmäßig mit den Beleuchtungsverhältnissen.

US 2015/031993 A1 beschreibt ein Verfahren zur Überwachung der Positionierung einer Intraokularlinse während einer chirurgischen Behandlung unter Verwendung von OCT.

Aufgabe der vorliegenden Erfindung ist es deshalb, Vorrichtungen und ein Verfahren zu beschreiben, welche die oben genannten Probleme gegenwärtig eingesetzter LVC-Systeme adressieren. Insbesondere ist es Aufgabe der Erfindung, Vorrichtungen und Verfahren zur einfachen und zuverlässigen Ausrichtung bzw. Zentrierung des Patientenauges zum LVC-System zu beschreiben.

Diese Aufgabe wird erfindungsgemäß gelöst durch Systeme und Verfahren mit den Merkmalen der jeweiligen unabhängigen Ansprüche. Vorteilhafte Ausgestaltungen sind in den Unteransprüchen und in der Beschreibung angegeben. Verfahren und Verfahrensschritte, die während einer chirurgischen Behandlung stattfinden, sind nicht Teil der beanspruchten Erfindung und sollen ausschließlich als Beispiel einer Anwendung dienen.

In einem ersten Aspekt betrifft die Erfindung ein Verfahren zur Ausrichtung eines Systems zur laserbasierten Fehlsichtigkeitskorrektur relativ zu einem zu behandelnden Patientenauge. Das Verfahren umfasst ein Bereitstellen von vorgegebenen pre-operativen Vermessungsdaten, welche zumindest vorbestimmte Strukturen des Patientenauges charakterisieren, wobei die vorbestimmten Strukturen einen zu behandelnden Teil des Patientenauges umfassen. Zudem umfasst das Verfahren ein Vermessen zumindest eines Teils der vorbestimmten Strukturen des Patientenauges mittels eines OCT-Systems unmittelbar vor einer Behandlung zur Fehlsichtigkeitskorrektur des Patientenauges und Bereitstellen von OCT-Vermessungsdaten. Außerdem umfasst das Verfahren ein Vergleichen der OCT-Vermessungsdaten und der vorgegebenen pre-operativen Vermessungsdaten und Bereitstellen von Vergleichsdaten, sowie ein Ermitteln einer Positionierung und/oder Orientierung des zu behandelnden Teils des Patientenauges relativ zum System und Ausrichten des Systems relativ zum Patientenauge unter Verwendung der ermittelten Position und/oder Orientierung des zu behandelnden Teils des Patientenauges.

In einem weiteren Aspekt betrifft die Erfindung ein System zur laserbasierten Fehlsichtigkeitskorrektur eines Patientenauges. Das System umfasst ein OCT-System zur Vermessung von Strukturen des Patientenauges unmittelbar vor und/oder während einer Behandlung zur Fehlsichtigkeitskorrektur des Patientenauges und zur Bereitstellung von OCT-Vermessungsdaten, welche die vermessenen Strukturen des Patientenauges charakterisieren. Zudem umfasst das System eine Steuereinheit, die dazu eingerichtet ist, die von dem OCT-System bereitgestellten OCT-Vermessungsdaten mit vorgegebenen pre-operativen Vermessungsdaten zu vergleichen und unter Verwendung von Vergleichsdaten aus dem Vergleich der vom OCT-System bereitgestellten OCT-Vermessungsdaten mit vorgegebenen pre-operativen Vermessungsdaten eine Positionierung und/oder Orientierung des zu behandelnden Teils des Patientenauges relativ zum System zu ermitteln und das System relativ zum Patientenauge unter Verwendung der ermittelten Position und/oder Orientierung des zu behandelnden Teils des Patientenauges auszurichten.

OCT steht dabei für optische Kohärenztomographie. Entsprechend ist ein OCT-System dabei ein System, das dazu ausgelegt ist, optische kohärenztomographische Messungen an einem Patientenauge durchzuführen. Ein OCT-Verfahren ist entsprechend ein Verfahren zur Durchführung einer OCT-Messung an einem oder mehreren Patientenaugen. Dass das LVC-System ein OCT-System aufweist, bedeutet dabei, dass das OCT-System einen Teil des LVC-System bildet und insbesondere diesem zuzurechnen ist.

Vorgegebene pre-operative Vermessungsdaten sind dabei Daten, welche bei einer Vermessung des Patientenauges vor der Behandlung der Fehlsichtigkeit und unabhängig von der Ausrichtung des Systems relativ zum Patientenauge ermittelt wurden. Die vorgegebenen pre-operative Vermessungsdaten können beispielsweise als Diagnosedaten vorliegen, d.h. Daten, die bei der Diagnose ermittelt wurden. Insbesondere können die pre-operativen Vermessungsdaten in einer Messung ermittelt werden bzw. worden sein, welche unabhängig vom System zur laserbasierten Fehlsichtigkeitskorrektur (LVC-System) durchgeführt wurde. Beispielsweise können die pre-operativen Vermessungsdaten mit einem vom LVC-System separaten OCT-System ermittelt worden sein. Beispielsweise können die pre-operativen Vermessungsdaten in Form von elektronischen Daten bereitgestellt werden. Beispielsweise kann das System eine Schnittstelle aufweisen, um vorgegebene pre-operative Vermessungsdaten zu empfangen und/oder abzurufen. Auch kann das System eine Datenbank mit vorgegebenen pre-operativen Vermessungsdaten aufweisen und/oder dazu ausgebildet sein, mit einer solchen Datenbank verbunden zu werden.

Die vorbestimmten Strukturen des Patientenauges sind dabei Strukturen des Patientenauges, welche für die Bestimmung und/oder Überprüfung der Positionierung und/oder Orientierung des Patientenauges geeignet sind und herangezogen werden sollen. Die Strukturen sind dabei dahingehend vorbestimmt, dass diese auch mit dem OCT-System des LVC-Systems vermessen werden, um miteinander vergleichbare Datensätze zu erhalten. Die vorbestimmten Strukturen sind dabei optional Strukturen im Inneren des Auges, welche mit OCT-Messungen charakterisierbar sind. Beispielsweise können die vorbestimmten Strukturen einen Teil oder die gesamte Vorderkammer des Auges umfassen, welche mittels OCT-Messungen charakterisiert werden kann. Die dabei gewonnenen Messdaten können sodann für eine Bestimmung und/oder Überprüfung der Positionierung und/oder Orientierung des Auges herangezogen werden.

Ein zu behandelnder Teil des Patientenauges ist dabei der Teil, welcher mittels des Systems zur laserbasierten Fehlsichtigkeitskorrektur behandelt wird oder für die Behandlung vorgesehen ist. Insbesondere kann der zu behandelnde Teil des Patientenauges die Hornhaut des Patientenauges oder einen Teil davon umfassen oder daraus bestehen. Insbesondere kann die Behandlung eine Ablation eines Teils der Hornhaut mittels des Systems umfassen und/oder ein Herauslösen eines Lentikels aus dem Inneren der Hornhaut.

Dass das Vermessen zumindest eines Teils der vorbestimmten Strukturen des Patientenauges mittels des OCT-Systems unmittelbar vor und/oder während einer Behandlung zur Fehlsichtigkeitskorrektur des Patientenauges erfolgt, bedeutet dabei, dass das Vermessen zeitgleich mit der Durchführung der Behandlung erfolgt und/oder in direktem zeitlichen Zusammenhang mit der Behandlung vor der Behandlung, insbesondere dann, wenn sich das Patientenauge bereits an der vorgesehen Position relativ zum System befindet, um die Behandlung durchzuführen. Optional ist nach dem Vermessen des zumindest einen Teils der vorbestimmten Strukturen des Patientenauges mittels des OCT-Systems keine Änderung der Positionierung und/oder Orientierung des Patientenauges relativ zum System erforderlich, welche über eine Zentrierung und/oder Ausrichtung des Systems hinausgeht. Das Bereitstellen von OCT-Vermessungsdaten bedeutet dabei, dass die Ergebnisse des Vermessens des zumindest einen Teils der vorbestimmten Strukturen des Patientenauges mittels des OCT-Systems unmittelbar vor und/oder während einer Behandlung zur Fehlsichtigkeitskorrektur des Patientenauges zumindest teilweise bereitgestellt werden, insbesondere in einem solchen Umfang, wie er für die Ausrichtung des Systems nützlich ist. Die OCT-Vermessungsdaten können damit Therapiedaten darstellen, die im Rahmen einer Therapie der Fehlsichtigkeitskorrektur ermittelt werden.

Das Bereitstellen von Vergleichsdaten bedeutet dabei, dass das Ergebnis des Vergleichs zumindest in solch einem Umfang dem System zur weiteren Verwendung zur Verfügung gestellt wird, wie dies für die Ausrichtung des Systems nützlich ist. Die Vergleichsdaten können optional eine Lateralverschiebung Δ*_{OCT}* und/oder einer Verdrehung Δ*β_{OCT}* der OCT-Messdaten um die optische Achse und/oder eine Verkippung Δ*α_{OCT}* ("Tip/Tilt") gegen die optische Achse gegenüber den vorgegebenen pre-operativen Messdaten umfassen.

Das Ausrichten des Systems stellt dabei ein Einstellen des Systems dar, sodass das System auf eine für die Behandlung der Fehlsichtigkeit gewünschte Position des Patientenauges zentriert ist. Das Ausrichten umfasst dabei optional keinen chirurgischen und therapeutischen Schritt. Das Ausrichten umfasst insbesondere ein Positionieren und/oder Orientieren des Systems derart, dass dieses auf die gewünschte Position des Auges, beispielsweise auf den Vertex oder eine davon abweichende Offset-Position, zentriert ist. Dabei kann das System oder ein Teil davon, wie etwa ein Anwendungsteil und/oder eine Abbildungsoptik bzw.

Fokussierungsoptik des Systems bewegt werden, um die gewünschte Orientierung und/oder Positionierung relativ zum Patientenauge einzunehmen. Alternativ oder zusätzlich kann der Patient bzw. das Patientenauge bewegt werden, um die gewünschte Positionierung und/oder Orientierung relativ zum Patientenauge einzunehmen.

Die Erfindung bietet den Vorteil, dass eine besonders präzise und zuverlässige Ausrichtung des Systems zur Ausrichtung eines Systems zur laserbasierten Fehlsichtigkeitskorrektur relativ zu einem Patientenauge ermöglicht wird. Durch die Verwendung von OCT-Vermessungsdaten, welche mittels des Systems vom Patientenauge gewonnen werden, und deren Vergleich mit vorgegebenen pre-operativen Vermessungsdaten wird eine äußerst präzise Überprüfung und/oder Anpassung der Positionierung und/oder Orientierung des Patientenauges ermöglicht.

Die Erfindung bietet weiterhin den Vorteil, dass die Ausrichtung unabhängig von individuellen Eigenschaften des Patientenauges mit großer Zuverlässigkeit erfolgen kann, insbesondere unabhängig von der Augenfarbe des Patientenauges und dem sich daraus ergebenden optischen Kontrast der Iris, sowie unabhängig von der Form der Hornhaut. Auch kann auf diese Weise bei der präzisen Ausrichtung die Zyklorotation des Auges zuverlässig erkannt und berücksichtigt werden, wodurch die Präzision und Zuverlässigkeit der Ausrichtung noch weiter verbessert werden kann.

Ferner bietet die Erfindung den Vorteil, dass diese in die allermeisten oder alle gängigen Systeme zur laserbasierten Fehsichtigkeitskorrektur implementiert werden kann, insbesondere in UV-Laser basierte System, welche etwa einen Excimerlaser aufweisen, und Pico- oder Femtosekundenlaser basierte Systeme.

Die Erfindung ermöglicht ferner eine neuen Form der tomographischen Zentrierung, die insbesondere in Verbindung mit einem am LVC-System optional eingesetzten Kontaktinterface und der eingesetzten Systemoptik und einem Abbildungsobjektiv direkt implantierbar und als visuelle Zentrierhilfe hilfreich ist.

Das Verfahren basiert auf dabei den vorgegebenen pre-operativen Vermessungsdaten und erlaubt eine Ausrichtung bzw. Zentrierung basierend beispielsweise auf der Vorderkammer-Tomographie, also nicht lediglich basierend auf der Hornhautoberfläche.

Damit werden weitere Ungenauigkeiten bei der Zentrierung von UVL-LVC-Systemen nach dem Stand der Technik adressiert. Allerdings ist ein solches System und Verfahren zur Referenzierung und Offset-Koordinaten-Bestimmung für das Scansystem, zum Beispiel durch eine automatische Korrektur oder eine manuelle Korrektur, nicht auf ein UV-Laser basiertes System zur Fehlsichtigkeitskorrektur beschränkt, sondern die erfindungsgemäßen Prinzipien sind auch für anderen System zur Fehlsichtigkeitskorrektur / zur Augenchirurgie anwendbar, wie beispielsweise Femto- und Pikosekunden-Lasersysteme.

Optional umfasst das Ausrichten des Systems ein Zentrieren des Systems auf den Vertex des Patientenauges. Optional umfasst das Ausrichten des Systems ein Zentrieren des Systems auf eine vom Vertex abweichende Offset-Position und optional ein Bestimmen von Offset-Koordinaten der Offset-Position. Dies bietet den Vorteil, dass der Benutzer bei Bedarf individuell die exakte Behandlungsposition des Patientenauges festlegen kann. Optional umfasst das Zentrieren des Systems auf die Offset-Position ein Berechnen einer zentrierungskorrigierten Fluence-Verlustfunktion. Dies bietet den Vorteil, dass die Ablation präzise eingestellt werden kann und eine etwaige Abweichung der Ablation von der Planung reduziert werden kann.

Die Ausrichtung des Systems kann optional manuell, teilweise automatisiert oder vollständig automatisiert erfolgen. Beispielsweise können systemabhängig unterschiedliche Grade der Automatisierung vorgesehen sein. Auch können Systeme gemäß manchen optionaler Ausführungsformen sowohl eine automatisierte, als auch eine teilautomatisierte und/oder manuelle Ausrichtung ermöglichen. Die manuelle Ausrichtung kann dabei durch das System unterstützt werden, insbesondere durch den erfindungsgemäßen Vergleich von OCT-Vermessungsdaten mit vorgegebenen pre-operativen Vermessungsdaten.

Optional umfassen die vorbestimmten Strukturen des Patientenauges, welche durch die vorgegebenen pre-operativen Vermessungsdaten charakterisiert sind, und der zumindest eine Teil der vorbestimmten Strukturen des Patientenauges, welcher mittels des OCT-Systems unmittelbar vor und/oder während einer Behandlung zur Fehlsichtigkeitskorrektur vermessen wird, zumindest einen Teil der Vorderkammer des Patientenauges. Dies bietet den Vorteil, dass anhand der Vorderkammer des Patientenauges eine zuverlässige Überprüfung ermöglicht wird und insbesondere eine eindeutige Zuordnung der Positionierung und/oder Orientierung der Vorderkammer zur Orientierung und/oder Positionierung des zu behandelnden Teils des Patientenauges, etwa der Hornhaut, möglich ist. Zudem bietet dies den Vorteil, dass der Messaufwand für das Vermessen der Vorderkammer gering gehalten werden kann und der für die Vermessung benötigte Zeitaufwand kurz gehalten werden kann. Optional umfasst der zu behandelnde Teil des Patientenauges zumindest einen Teil der Hornhaut.

Optional umfasst das Verfahren ferner ein Koppeln des Patientenauges an ein Kontaktinterface des Systems zur laserbasierten Fehlsichtigkeitskorrektur. Optional umfasst das System dazu ferner ein Kontaktinterface zur Kopplung des Patientenauges an das System. Dies ermöglicht ein zuverlässiges Fixieren des Patientenauges relativ zum System. Dabei erfolgt das Vermessen des zumindest einen Teils der vorbestimmten Strukturen des Patientenauges mittels des OCT-Systems optional zumindest einmal vor dem Koppeln des Patientenauges an das Kontaktinterface und zumindest einmal nach dem Koppeln des Patientenauges an das Kontaktinterface. Dies bietet den Vorteil, dass etwaige Änderungen der Positionierung und/oder Orientierung des Patientenauges durch das Andocken des Kontaktinterfaces, wie beispielsweise eines Kontaktglases, erkannt und kompensiert und/oder bei der Behandlung berücksichtigt werden können.

Optional umfasst das Verfahren ferner ein Überprüfen der Positionierung und/oder Orientierung des zu behandelnden Teils des Patientenauges relativ zum System mittels eines Eye-Trackers, wobei das Überprüfen optional fortlaufend erfolgt. Optional umfasst das System dazu ferner einen Eye-Tracker zur Überprüfung der Position und/oder Orientierung des Patientenauges relativ zum System, insbesondere relativ zur Abbildungsoptik. Dies bietet den Vorteil, dass während der Behandlung etwaige Änderungen der Positionierung und/oder Orientierung des Patientenauges zuverlässig erkannt und ggf. kompensiert und/oder anderweitig bei der Behandlung berücksichtigt werden können. Insbesondere bietet die Verwendung eines Eye-Trackers den Vorteil, dass kein Fixieren des Patientenauges erforderlich ist und entsprechend auch für solche Verfahren eine zuverlässige Ausrichtung und/oder Positionierung erfolgen kann, welche kein Fixieren des Patientenauges mittels eines Kontaktinterfaces beinhalten.

Das System kann ferner dazu eingerichtet sein, ein Referenzieren einer mittels des Eye-Trackers bestimmten Augenposition (beispielsweise eine Pupillenposition und/oder Limbusposition) unter Verwendung der OCT-Vermessungsdaten vorzunehmen. Dies bietet den Vorteil, dass mittels des Eye-Trackers eine fortlaufende Überwachung der Positionierung und/oder Orientierung des Patientenauges ermöglicht wird und zudem durch eine initiale Referenzierung vor der Behandlung und/oder regelmäßige Referenzierung während der Behandlung (etwa in Behandlungspausen) ein hohes Maß an Präzision erreicht werden kann.

Optional umfassen die vorgegebenen pre-operativen Vermessungsdaten pre-operative OCT-Vermessungsdaten. Dies bietet den Vorteil, dass ein hohes Maß an Vergleichbarkeit zwischen den pre-operativen Vermessungsdaten und den durch das System bereitgestellten OCT-Vermessungsdaten erreicht werden kann.

Optional umfassen die Vergleichsdaten eine Lateralverschiebung und/oder eine Verdrehung und/oder eine Verkippung der OCT-Vermessungsdaten relativ zu den pre-operativen OCT-Vermessungsdaten. Dadurch kann mit hoher Präzision und Zuverlässigkeit eine etwaige Abweichung der Positionierung und/oder Orientierung des Patientenauges von der Sollposition erkannt und/oder quantifiziert werden.

Die Verdrehung kann dabei beispielsweise eine Verdrehung des Patientenauges gemäß den OCT-Vermessungsdaten relativ zum Patientenauge gemäß den pre-operativen Vermessungsdaten darstellen. Die Verdrehung kann beispielsweise als eine Verdrehung um eine Zentralachse bzw. visuelle Achse des System und/oder einer Optik des Systems und/oder als eine Verdrehung um eine optische Achse des Auges gemäß den pre-operativen Vermessungsdaten, die mittig durch die Pupille verläuft, bestimmt werden. Alternativ oder zusätzlich kann die Verdrehung als eine Verdrehung um die keratometrische Achse des Patientenauges gemäß den pre-operativen Vermessungsdaten, die durch den Vertex verläuft, bestimmt werden.

Alternativ oder zusätzlich kann eine Verkippung des Auges gemäß den OCT-Vermessungsdaten relativ zum Auge gemäß den pre-operativen Vermessungsdaten in einer oder mehreren, insbesondere zwei, Richtungen bestimmt werden. Zur Bestimmung der Verkippung kann dabei beispielsweise eine Verkippung der optischen Achse des Auges und/oder der keratometrischen Achse des Auges herangezogen werden, welche aus den Vergleichsdaten bestimmt werden kann.

Optional umfasst das System eine Laserquelle zur Bereitstellung einer Laserstrahlung für die Behandlung, wobei die Laserquelle optional dazu ausgelegt ist, gepulste Laserstrahlung zu emittieren und wobei die Laserquelle einen Excimer-Laser und/oder einen Picosekundenlaser und/oder einen Femtosekundenlaser umfasst oder als Excimer-Laser, Picosekundenlaser, Femtosekundenlaser ausgebildet ist. Je nach der angestrebten Behandlungstechnik kann somit das System mit einer dafür geeigneten Laserquelle ausgestattet sein. Gemäß manchen optionalen Ausführungsformen kann das System sowohl eine UV-Laserquelle als auch einen Piko- und/oder Femtosekundenlaser aufweisen. Dies ermöglicht, sowohl Ablations-basierte Verfahren als auch Verfahren zur Entfernung eines Lentikels durchzuführen.

Optional umfasst das System ferner eine Abbildungsoptik zur Fokussierung der Laserstrahlung auf die Hornhaut des Patientenauges, wobei die Abbildungsoptik derart ausgestaltet ist, dass die Abbildungsoptik ein Erfassen eines Rückreflexes von Strahlung, die durch die Abbildungsoptik auf das Patientenauge eingestrahlt und zumindest teilweise vom Patientenauge reflektiert wird, in einem Akzeptanzwinkel *χ_{Max}* von mindestens 2,5° ermöglicht. Dies bietet den Vorteil, dass der Rückreflex der Strahlung, die durch die Abbildungsoptik auf das Patientenauge eingestrahlt und zumindest teilweise vom Patientenauge reflektiert wird, in einem besonders großen Akzeptanzwinkel aufgesammelt für die Analyse dem System bereitgestellt werden kann. Dies erleichtert die Durchführung der OCT Vermessung der vorbestimmten Strukturen des Patientenauges und erleichtert somit die Ausrichtung des Systems relativ zum Patientenauge. Optional umfasst das Erfassen des Rückreflexes von Strahlung ein Erfassen eines Rückreflexes eines durch die Abbildungsoptik in das Patientenauge eingestrahlten OCT-Strahls.

Dass die Abbildungsoptik ein Erfassen des Rückreflexes in einem Akzeptanzwinkel *χ* Max von mindestens 2,5° ermöglicht, bedeutet dabei, dass der Akzeptanzbereich für Strahlen des Rückreflexes mit einem Winkel zur optischen Achse der Abbildungsoptik erfasst. Eine Abbildungsoptik mit einem Akzeptanzwinkel von 2,5° kann demnach beispielsweise optische Strahlen des Rückreflexes erfassen, welche in einem Lichtkegel mit Öffnungswinkel von 5° zur Einfallsrichtung (also mit einem Öffnungswinkel der Mantelfläche des Lichtkegels zur Mittelgeraden des Lichtkegels von 2,5°) von der Hornhaut zur Abbildungsoptik reflektiert werden, sodass diese von der Abbildungsoptik aufgesammelt und vom System verwendet werden können.

Optional ist die Abbildungsoptik derart ausgebildet, dass der Akzeptanzwinkel *χ_{Max}* größer als 5°, optional größer als 10°, optional größer als 15°, optional größer als 25° und optional größer oder gleich 37° ist. Dies vergrößert den Bereich, in dem die vom Auge reflektierte Strahlung aufgesammelt und für die OCT-Vermessung verwendet werden kann. Somit wird dadurch der vermessbare Bereich des Patientenauges vergrößert und die Präzision und Zuverlässigkeit der Ausrichtung verbessert.

Optional ist die Abbildungsoptik als eine Mikroskop-Optik ausgebildet oder umfasst eine solche. Die Abbildungsoptik weist dabei optional eine optische Öffnung und einen vorgegebenen Arbeitsabstand auf, bei welchem ein Durchmesser der optischen Öffnung größer als oder gleich dem vorgegebenen Arbeitsabstand ausgebildet ist. Optional weist die Abbildungsoptik eine optische Öffnung mit einem Durchmesser von zumindest 50 mm, optional von zumindest 60 mm, auf. Die Abbildungsoptik weist ferner optional einen Arbeitsabstand auf der kleiner als 50 mm und optional kleiner oder gleich 40 mm ist. Dies bietet den Vorteil, dass ein besonders großer Akzeptanzwinkel bereitgestellt werden kann.

Die oben genannten und im Folgenden erläuterten Merkmale und Ausführungsformen sind dabei nicht nur als in den jeweils explizit genannten Kombinationen offenbart anzusehen, sondern sind auch in anderen technisch sinnhaften Kombinationen und Ausführungsformen vom Offenbarungsgehalt umfasst.

Weitere Einzelheiten und Vorteile der Erfindung sollen nun anhand von den folgenden Beispielen und bevorzugten Ausführungsformen mit Bezug auf die Figuren näher erläutert werden.

Es zeigen:
- Figur 1:: eine schematische Darstellung eines ungeeignet platzierten Ablationsprofils;
- Figur 2:: eine schematische Darstellung eines LVC-Systems gemäß einer optionalen Ausführungsform;
- Figur 3A:: einen Strahlenverlauf eines LVC-Systems gemäß einer optionalen Ausführungsform;
- Figur 3B:: einen beispielhaften Akzeptanzwinkel eines herkömmlichen LVC-Systems;
- Figur 3C:: einen beispielhaften Akzeptanzwinkel eines LVC-Systems gemäß einer optionalen Ausführungsform;
- Figur 4:: Visualisierungen von OCT-Messdaten und vorgegebenen pre-operativen Vermessungsdaten zur Erläuterung des Prinzips eines Verfahrens zur Ausrichtung eines LVC-Systems relativ zu einem Patientenauge gemäß einer optionalen Ausführungsform.

In den folgenden Figuren werden gleiche oder ähnliche Elemente in den verschiedenen Ausführungsformen der Einfachheit halber mit gleichen Bezugszeichen bezeichnet.

Figur 1 zeigt in einer schematischen Darstellung das Prinzip der Entstehung prismatischer Korrekturfehler (Tip/Tilt) gemäß eines herkömmlichen Zentrierverfahrens, wenn der Patient nicht auf das Zentrum des Fixierobjekts bzw. der "Fixationswolke" fixiert. Dabei ist in Figur 1 ein Patientenauge 10 mit Cornea 12 und Fovea 14 dargestellt, sowie die optische Achse 16 des Patientenauges bzw. die visuelle Achse 16, ein Ablationsprofil 18, ein Scansystem 20 und ein Fixierelement 22, auf welches der Patient den Blick zu fixieren hat. Die visuelle Achse 16 schneidet die Hornhaut 12 im ophthalmischen Pol 24 (engl.: ophthalmic pole; Schnittpunkt der visuellen achse mit der Hornhautvorderfläche unter Patientenfixation).

Fixiert der Patient das Auge 10 nicht wie vorgesehen auf das Zentrum des Fixierelements 22, sondern beispielsweise auf einen Randbereich des Fixierelements 22, kann dies zur Folge haben, dass das Ablationsprofil 18 nicht korrekt entlang der notwendigen Behandlungsachse appliziert wird (z.B. entlang der visuellen Achse 16; definiert durch den ophthalmischen Pol (OP) und den vom Patientenauge 10 fixierten Punkt des Fixierelements 22 und damit nicht orthogonal zur visuellen Achse 16). Die Verhältnisse sind in Figur 1 zur besseren Veranschaulichung stark übertrieben dargestellt.

Bei der Behandlung eines Patientenauges mit einem LVC-System ist daher sicherzustellen, dass die Erzeugung derartiger prismatischer Fehler vermieden werden. Wird dies nicht sichergestellt, kann der unerwünschte Fall eintreten, dass die Ablationsprofile 18 nicht in der korrekten Ebene, d.h. nicht auf die Oberflächennormale, also senkrecht zur visuellen Achse 16, appliziert werden. Dies kann dadurch begünstigt werden, dass der Patient bevorzugt in eine weitgehend feste, aber "falsche" Richtung fixiert, also z.B. permanent in eine feste Richtung schaut, die nicht dem Zentrum des Fixierelements 22 entspricht (während der Operation kann der Patient das Fixationstarget je nach Refraktionsdefizit und Behandlungsdauer nicht mehr scharf sehen).

Figur 2 zeigt in einer schematischen Darstellung ein LVC-System 100 gemäß einer optionalen Ausführungsform der Erfindung. Das LVC-System 100 weist eine Laserquelle 102, ein Scansystem 104, eine Steuereinheit 106 sowie eine Planungseinheit 108 und ein OCT-System 109 auf. Zum Datenaustausch der Steuereinheit 106 mit der Laserquelle 102, dem Scanner 104 und der Planungseinheit 108 sowie mit dem OCT-System 109 weist die Steuereinheit 106 Schnittstellen (als Kästen an der Steuereinheit S dargestellt) auf, über die die Datenleitung über Kabel übertragen werden können. Die Planungseinheit 108 weist ebenfalls eine Schnittstelle (als Kasten an der Planungseinheit P dargestellt) auf für einen Datenaustausch mit der Steuereinheit 106. Eine kabellose Übertragung ist ebenfalls möglich. Die Planungseinheit 108 weist eine Recheneinheit auf (nicht dargestellt), über die die Planungsdaten berechnet werden. Die Begriffe Scanner und Scansystem werden im Rahmen dieser Offenbarung als Synonyme verwendet.

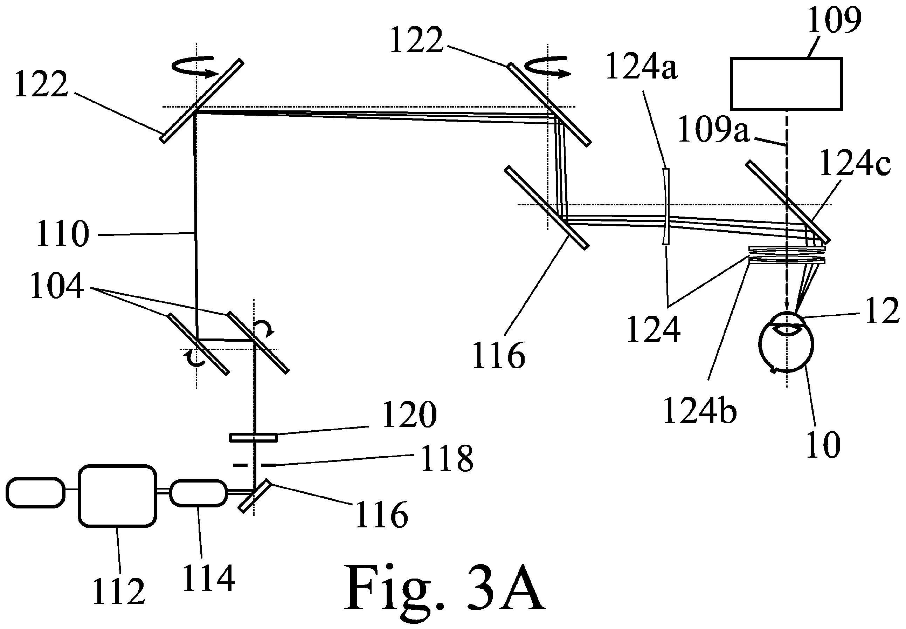

In Figur 3A ist beispielhaft eine Prinzip-Anordnung des optischen Strahlengangs eines Ausführungsbeispiels eines LVC-Systems 100, insbesondere des Anwendungsteils des LVC-Systems 100, gezeigt. Ein Laserstrahl 110 wird von einem UV-Laser oder einem Femtosekundenlaser oder einem Picosekundenlaser 112 als Laserquelle bereitgestellt. Der Laserstrahl 110 wird von einem (optionalen) optischen Abschwächer ("optical attenuator") 114 abgeschwächt, von einem Umlenker 116 umgelenkt, fällt auf eine Blende (oder ein Pinhole) 118 und gelangt anschließend in das Strahlformungselement ("beam shaper") 120. Dieser dient der Strahlformung des Laser Rohstrahls in eine Gauss oder Super-Gauß Pulse-Fluence Verteilung. Über das Scansystem 104 kann der Laserstrahl 110 lateral in x- und y-Richtung abgelenkt werden (dargestellt über gebogene Pfeile). Von hier aus wird der Laserstrahl 110 in einen ersten Gelenkarm geführt. Dieser ist im gezeigten Ausführungsbeispiel mit einer Grundeinheit (nicht eingezeichnet) über ein erstes Drehgelenk 122 (symbolisch dargestellt über eine Drehachse und einen Drehpfeil) beweglich verbunden. Die Grundeinheit weist die Laserquelle 102 bzw. den Excimer-Laser 112, den optischen Abschwächer 114, die Blende 118 (sowie den Umlenker 116, der sich im Strahlengang zwischen dem optischen Abschwächer 114 und der Blende 118 befindet), das Strahlformungselement 120 sowie das Scansystem 104 auf. Der erste Gelenkarm ist auf der der Grundeinheit abgewandten Seite über ein zweites Drehgelenk 122 (symbolisch dargestellt über eine Drehachse und einen Drehpfeil) mit einem zweiten Gelenkarm beweglich verbunden. Der Laserstrahl 110 wird durch das zweite Drehgelenk 112 über zwei weitere Umlenker 116 in den zweiten Gelenkarm geführt. Gemäß weiteren optionalen Ausführungsformen können zusätzlich noch weitere Drehgelenke ausgebildet sein. Von dort wird der Laserstrahl 110 in Richtung Patientenauge 10 über einen weiteren Umlenker 116 gelenkt. Dabei wird der Laserstrahl 110 über eine Fokussieroptik bzw. Abbildungsoptik 124 auf die Hornhaut 12 des Patientenauges 10 fokussiert. Dabei ist die Abbildungsoptik 124 zweiteilig aufgebaut. Zwischen der ersten Linsengruppe 124a und der zweiten Linsengruppe 124b befindet sich im Strahlengang ein Umlenker 124c. Die erforderlichen Linsen der beiden Linsengruppen 124a, 124b sind nur schematisch dargestellt. Die Abbildungsoptik 124 ist dabei derart ausgebildet, dass diese einen Akzeptanzwinkel zum Erfassen eines Rückreflexes einer durch die Abbildungsoptik auf die Hornhaut eingestrahlten Strahlung von mindestens 2,5° aufweist. Gemäß der gezeigten Ausführungsform dient der Umlenker auch dazu, einen OCT-Laserstrahl 109a über die Abbildungsoptik 124 in das Patientenauge einzukoppeln und den reflektierten und von der Abbildungsoptik 124 aufgesammelten Rückreflex zum OCT-System 109 zurückzuleiten.

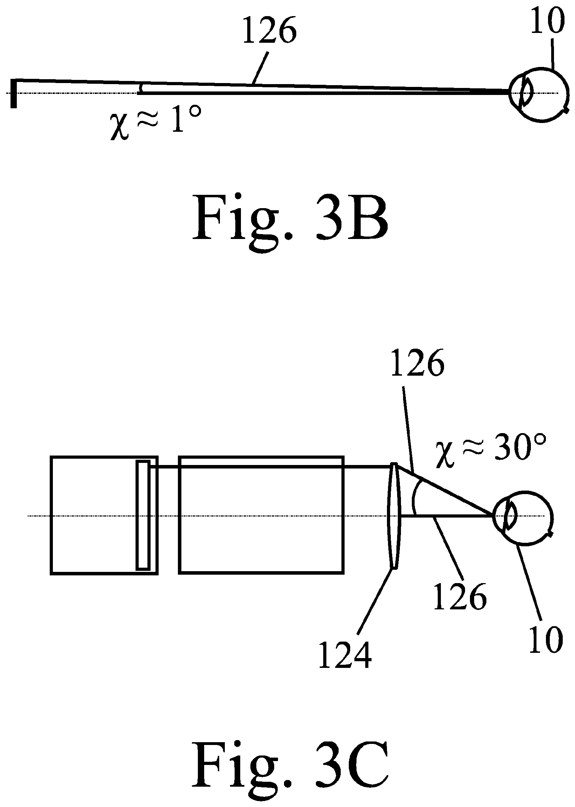

Die Figuren 3B und 3C veranschaulichen in schematischen Darstellungen ein herkömmliches LVC-System (Figur 3B) mit einem Akzeptanzwinkel für Rückreflexe 126 von etwa 1° im Vergleich zu einem LVC-System gemäß einer optionalen Ausführungsform der Erfindung mit einem Akzeptanzwinkel von etwa 30°.

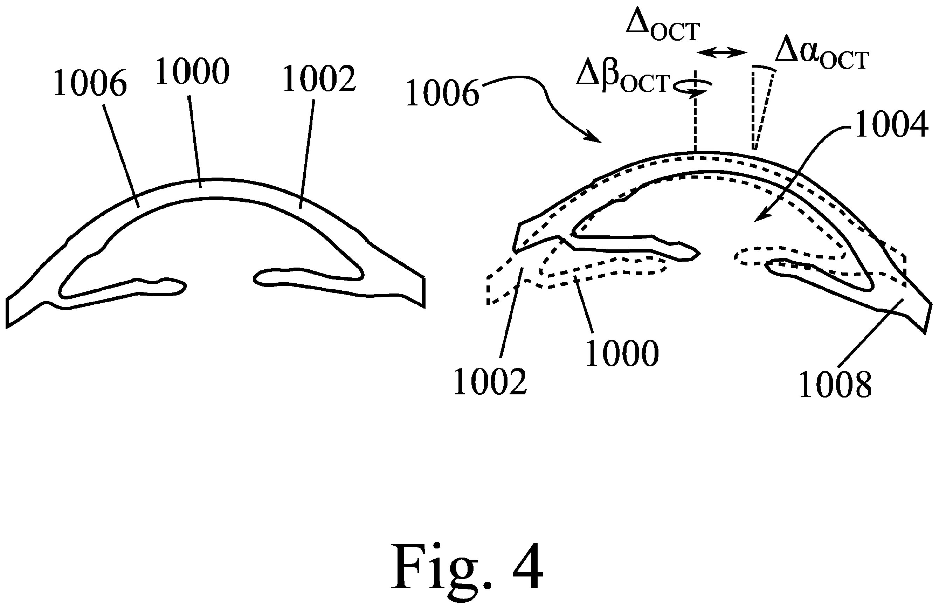

Mit Verweis auf Figur 4 wird im Folgenden das Prinzip eines Verfahrens zur Ausrichtung eines LVC-Systems relativ zu einem Patientenauge gemäß einer optionalen Ausführungsform beschrieben.

Das linke Teilbild zeigt dabei schematisch und beispielhaft vorgegebene pre-operative Vermessungsdaten 1000 von vorbestimmten Strukturen 1002 des Patientenauges 10. Die vorbestimmten Strukturen umfassen dabei die Vorderkammer 1004 des Patientenauges 10 und auch den zu behandelnden Teil 1006 des Patientenauges 10, welcher gemäß der erläuterten optionalen Ausführungsform einen Teil der Hornhaut 12 des Patientenauges 10 umfasst.

Die Algorithmen des LVC-Systems gemäß der optionalen Ausführungsform erkennen dabei die Kontur der Vorderkammer (Umrandung in den Teilbildern der Figur 4). Diese Kontur dient sodann bei der Ausrichtung bzw. Zentrierung des LVC-Systems relativ zum Patientenauge, insbesondere bei Einsatz eines Kontaktinterfaces bei der Kontaktinterface-Adaption an das Patientenauge als Referenz (OCT Referenzkontur) für die Kontaktinterface-Positionierung und Zentrierung.

Die vorgegebenen pre-operativen Vermessungsdaten 1000 dienen dabei als Referenzdaten und werden durch ein geeignetes Diagnosegerät bereitgestellt, beispielsweise mittels eines vom LVC-System unabhängigen OCT-Systems (z.B. MS 39 CSO, nicht gezeigt). Die vorgegebenen pre-operativen Vermessungsdaten 1000 können dabei als pre-operative OCT-Vermessungsdaten 1000 bereitgestellt werden und werden für die Ausrichtung des LVC-Systems 100 verwendet.

Sofern die optionale Ausführungsform ein Koppeln und Fixieren des Patientenauges an ein Konttaktinterface vorsieht, können die pre-operativen OCT-Vermessungsdaten als Referenz für die Kontaktinterface-Positionierung und Zentrierung verwendet werden. Die pre-operativen OCT-Vermessungsdaten werden hierfür dem LVC-System bereitgestellt.

Beispielsweise kann das LVC-System anhand der pre-operativen OCT-Vermessungsdaten eine Bestimmung und Verwendung der OCT Kontur der Vorderkammer und/oder der Hornhaut (siehe Konturlinie) vorsehen. Gemäß der erläuterten Ausführungsform vermisst sodann das LVC-System mittels eines eigenene OCT-Systems zumindest einen Teil der vorbestimmten Strukturen des Patientenauges unmittelbar vor und/oder während einer Behandlung zur Fehlsichtigkeitskorrektur des Patientenauges und stellt entsprechende OCT-Vermessungsdaten 1008 bereit. Optional erfolgt dies mit identischer OCT-Technologie bzw. in gleicher Art und Weise in-situ, also kurz vor und nach erfolgter Kontaktinterface-Adaption, wobei OCT-Messungen wiederholend vorgenommen werden und ggf. ebenso die OCT-Kontur bestimmt. Das systemeigene OCT-System kann entsprechend ähnlich oder identisch zu dem OCT-System ausgebildet sein, mit welchem die vorgegebenen pre-operativen Vermessungsdaten 1000 ermittelt wurden.

Sodann erfolgt durch das LVC-System ein Vergleichen der OCT-Vermessungsdaten und der vorgegebenen pre-operativen Vermessungsdaten und ein Bereitstellen von Vergleichsdaten. Darüber hinaus erfolgt durch das LVC-System ein Ermitteln einer Positionierung und/oder Orientierung des zu behandelnden Teils des Patientenauges relativ zum System und Ausrichten des Systems relativ zum Patientenauge unter Verwendung der ermittelten Position und/oder Orientierung des zu behandelnden Teils des Patientenauges. Das Ausrichten kann dabei vollständig oder teilweise automatisiert durch das LVC-System erfolgen oder zumindest teilweise manuell durch den Anwender.

Für Zwecke der manuellen Ausrichtung bzw. Zentrierung bezüglich der OCT-Daten werden dem Anwender die vorgegebenen pre-operativen Vermessungsdaten 1000 und jene der aktuellen (in-situ) OCT-Messung durch das LVC System, d.h. die OCT Vermessungsdaten, auf einem Display visualisiert und zwar bezüglich einer gleichen Koordinaten-Referenz ("Referenz-Koordinatensystem"). Dies ist im rechten Teilbild der Figur 4 dargestellt. Durch eine geeignete Anwendungssoftware können die vorgegebenen pre-operativen Vermessungsdaten und die OCT-Vermessungsdaten 1008 optional in überlagerter Weise zusammen als zwei Layer dargestellt werden, um dem Anwender die manuelle Ausrichtung und/oder die Überwachung der automatisierten Ausrichtung zu erleichtern.

Zur manuellen Ausrichtung bzw. Zentrierung kann der Anwender die Optik zur Applikation der Laserstrahlung auf das Patientenauge, welche beispielsweise als eine Mikroskop-Optik ausgebildet sein kann oder eine solche umfassen kann, über dem Auge lateral so in der x,y Ebene verschieben (etwa durch eine Verschiebung eines Anwendungsteils, in welches die Optik integriert ist), dass die beiden OCT-Bilder, d.h. die visuelle Darstellung der vorgegebenen pre-operativen Vermessungsdaten 1000 und der OCT-Vermessungsdaten 1008, eine möglichst geringe Abweichung in ihrer Positionierung und/oder Orientierung voneinander aufweisen. Zur weiteren Unterstützung des Anwenders können auf einem Display zur Visualisierung optional Richtungs-Indikatoren dargestellt werden oder z.B. die Anzeige einer Lateralverschiebung Δ*_{OCT}* und/oder einer Verdrehung Δ*α_{OCT}* der Visualisierung der OCT-Vermessungsdaten 1008 gegenüber den vorgegebenen pre-operativen Vermessungsdaten 1000. Unter Patientenfixation sollten bei der Lateralverschiebung des Anwendungsteils in der x,y Ebene zur manuellen Zentrierung beide Größen, d.h. die Lateralverschiebung Δ*_{OCT}* und/oder einer Verdrehung Δ*α_{OCT}* und/oder einer Verkippung, zusammen gegen Null gehen mit Verbesserung der Zentrierung.

Um auch die Verwendung von OCT-Systemen mit einer begrenzten Bandbreite zu ermöglichen, kann die Wiederholrate der Daten zum Abgleich mit den vorher erfassten Daten eingeschränkt werden. Insbesondere kann der OCT Scanvorgang optional nach Sichtung und Bewertung der Diagnosedaten zwischenzeitlich auf eine charakteristischen Teilbereich der Koordinaten begrenzt stattfinden. Hierzu ist zunächst eine komplette Erfassung der Vorderkammer vorteilhaft und eine Detektion der charakteristischen vorausgewählten Teilbereiche, d.h. der vorgegebenen pre-operativen Vermessungsdaten, nötig. Dies kann manuell, teilweise automatisiert oder vollständig automatisiert über geeignete Verfahren der Bilderkennung geschehen. Fortan kann sodann auf eine erneute vollständige OCT-Vermessung der gesamten Vorderkammer verzichtet werden und stattdessen lediglich Teilbereiche erfasst und zwischen Diagnosedaten und Therapiedaten, d.h. zwischen den vorgegebenen pre-operativen Vermessungsdaten und OCT-Vermessungsdaten, abgeglichen werden. Da dies die Anzahl der erforderlichen B-Scans stark reduzieren kann, ist dies vorteilhaft für die Bandbreite der Therapiedatenerfassung und entsprechend für den Einsatz von OCT-Systemen mit reduzierter Bandbreite. Nach erfolgreicher Zentrierung der Teilbereichs-Daten zueinander kann zur finalen Kontrolle optional nochmals der langsame Scan aller Strukturen erfolgen.

Gemäß einer weiteren optionalen Ausführungsform umfasst das Verfahren die automatisierte Bereitstellung von Offset-Koordinaten aus der Lateralverschiebung Δ*_{OCT}* und der Verdrehung Δ*β_{OCT}* und der Verkippung Δ*α_{OCT}* und optional einer Verkippung. Dabei wird zur Ausrichtung bzw. Zentrierung des LVC-Systems auf einen z.B. vom Vertex und/oder dem Pupillenmittelpunkt der Hornaut abweichenden Punkt ein Satz von Offset-Koordinaten generiert, um etwa unter Verwendung dessen eine zentrierungskorrigierte Fluence-Verlustfunktion zu berechnen. Dies bietet die Möglichkeit, eine etwaige Abweichung zwischen der beabsichtigten und der tatsächlichen Ablation der Hornhaut zu reduzieren.

In einer weiteren optionalen Ausführungsvariante kann der Vergleich der OCT-Vermessungsdaten und der vorgegebenen pre-operativen Vermessungsdaten auch in einem kontaklosen Verfahren ohne Kontaktinterface angewendet werden, z.B. optional in Kombination mit einem Eye-Tracking System. Hierbei besteht die Möglichkeit, den OCT Strahl über das Tracking Signal des Eye-Trackers mitzuführen, um etwaige leichte Augenbewegungen auszugleichen. Alternativ kann auf das Mitführen des OCT Strahls verzichtet werden, wenn die OCT B-Scan Daten unter Verwendung der simultan erfassten Eyetracking-Koordinaten korrigiert werden.

In beiden Varianten kann dann das per OCT Vermessung bzw. Tomographie bestimmte Zentrum, d.h. der Punkt, auf welchen das System mittels der Ausrichtung zentriert wurde, auf das Pupillen- oder vorzugsweise Limbuszentrum und/oder die Iris referenziert werden und dieses fortan per Eyetracker mit hoher Bandbreite während des Ablationsvorgangs nachgeführt werden.

Das OCT System kann zudem in diesem Falle optional auch dazu verwendet werden, nach der tomographischen Zentrierung den korrekten z-Abstand zwischen Hornhaut und Lasersystem zu überwachen. Dies ist insbesondere dann von Vorteil, wenn aus anatomischen, medizinischen oder sonstigen Gründen ein Kontaktinterface nicht verwendet werden kann. Da im konvergenten Fokalfeld eine Augenverschiebung entlang der Symmetrieachse der Optik die Auftreffpunkte des Behandlungslichts stark beeinflusst, ist eine Detektion dieser Verschiebung in diesem Fall vorteilhaft. Eine Messung der z-Koordinate kann dann beispielsweise per 2D Eyetracking auf der Koordinate des Behandlungszentrums durchgeführt werden. Dies ist im Falle eines separaten Scansystems für den OCT Strahl sehr einfach und mit hoher Bandbreite möglich. Im Falle einer OCT Strahlführung über die Scanner des Behandlungslasers ist optional eine intermediäre Überprüfung der z-Position zwischen vereinzelten Schüssen oder statistisch zu den Zeitpunkten, wenn Abtragspulse ins Behandlungszentrum geschossen werden, möglich. Als Messsignal kann die Oberfläche der Hornhaut im Behandlungszentrum oder vorzugsweise die von der Behandlung nicht beeinträchtigte Grenzfläche zwischen Endothel und Kammerwasser auf der Rückseite des Behandlungszentrums dienen.

Die vorstehend genannten und in verschiedenen Ausführungsbeispielen erläuterten Merkmale der Erfindung sind dabei nicht nur in den beispielhaft angegebenen Kombinationen, sondern auch in anderen Kombinationen oder allein einsetzbar, ohne den Rahmen der vorliegenden Erfindung zu verlassen.

Eine auf Verfahrensmerkmale bezogene Beschreibung einer Vorrichtung gilt bezüglich dieser Merkmale analog für das entsprechende Verfahren, während Verfahrensmerkmale entsprechend funktionelle Merkmale der beschriebenen Vorrichtung darstellen.

### Bezugszeichenliste

- 10: Patientenauge
- 12: Cornea / Hornhaut
- 14: Fovea
- 16: visuelle Achse / optische Achse des Auges
- 18: Ablationsprofil
- 20: Scansystem
- 22: Fixierelement
- 24: ophtlamischer Pol

- 100: (LVC-)System
- 102: Laserquelle
- 104: Scanner bzw. Scansystem
- 106: Steuereinheit
- 108: Planungseinheit
- 109: OCT-System
- 109a: OCT-Laserstrahl bzw. OCT-Strahl
- 110: Laserstrahl
- 112: Laser
- 114: Abschwächer
- 116: Umlenker
- 118: Blende
- 120: Strahlformungselement
- 122: Drehgelenk
- 123: Anwendungsteil
- 124: Abbildungsoptik
- 124a: erste Linsengruppe der Abbildungsoptik
- 124b: zweite Linsengruppe der Abbildungsoptik
- 124c: Umlenker
- 126: Rückreflex
- *χ*: Öffnungswinkel des Rückreflexes
- *χ_{Max}*: maximal erfassbarer Öffnungswinkel des Rückreflexes bzw. Akzeptanzwinkel der Abbildungsoptik

- 1000: (Visualisierung der) pre-operativen Vermessungsdaten
- 1002: vorbestimmte Strukturen
- 1004: Vorderkammer des Patientenauges
- 1006: zu behandelnder Teil des Patientenauges
- 1008: (Visualisierung der) OCT-Vermessungsdaten
- Δ*_{OCT}*: Lateralverschiebung
- Δ*α_{OCT}*: Verkippung
- Δ*β_{OCT}*: Verdrehung

## Patentansprüche

1. Verfahren zur Ausrichtung eines Systems (100) zur laserbasierten Fehlsichtigkeitskorrektur relativ zu einem zu behandelnden Patientenauge (10), das Verfahren umfassend:
- Bereitstellen von vorgegebenen pre-operativen Vermessungsdaten (1000), welche zumindest vorbestimmte Strukturen (1002) des Patientenauges (10) charakterisieren, wobei die vorbestimmten Strukturen (1002) einen zu behandelnden Teil des Patientenauges (10) umfassen;
- Vermessen zumindest eines Teils der vorbestimmten Strukturen (1002) des Patientenauges (10) mittels eines OCT-Systems (109) unmittelbar vor einer Behandlung zur Fehlsichtigkeitskorrektur des Patientenauges (10) und Bereitstellen von OCT-Vermessungsdaten (1008), welche die vermessenen Strukturen des Patientenauges charakterisieren;
- Vergleichen der OCT-Vermessungsdaten (1008) und der vorgegebenen pre-operativen Vermessungsdaten (1000) und Bereitstellen von Vergleichsdaten aus dem Vergleich der vom OCT-System bereitgestellten OCT-Vermessungsdaten mit den vorgegebenen pre-operativen Vermessungsdaten;
**dadurch gekennzeichnet, dass** das Verfahren ferner aufweist:
- Ermitteln einer Positionierung und/oder Orientierung des zu behandelnden Teils des Patientenauges (10) relativ zum System (100) und Ausrichten des Systems (100) relativ zum Patientenauge (10) unter Verwendung der ermittelten Position und/oder Orientierung des zu behandelnden Teils des Patientenauges (10).

2. Verfahren gemäß Anspruch 1, wobei das Ausrichten des Systems (100) ein Zentrieren des Systems (100) auf den Vertex des Patientenauges (10) umfasst.

3. Verfahren gemäß Anspruch 1, wobei das Ausrichten des Systems (100) ein Zentrieren des Systems (100) auf eine vom Vertex abweichende Offset-Position und optional ein Bestimmen von Offset-Koordinaten der Offset-Position umfasst, und wobei das Zentrieren des Systems (100) auf die Offset-Position optional ein Berechnen einer zentrierungskorrigierten Fluence-Verlustfunktion umfasst.

4. Verfahren gemäß einem der vorhergehenden Ansprüche, wobei die Ausrichtung des Systems (100) manuell, teilweise automatisiert oder vollständig automatisiert erfolgt.

5. Verfahren gemäß einem der vorhergehenden Ansprüche, wobei die vorbestimmten Strukturen (1002) des Patientenauges (10), welche durch die vorgegebenen pre-operativen Vermessungsdaten (1000) charakterisiert sind, und der zumindest eine Teil der vorbestimmten Strukturen (1002) des Patientenauges (10), welcher mittels des OCT-Systems (109) unmittelbar vor und/oder während einer Behandlung zur Fehlsichtigkeitskorrektur vermessen wird, zumindest einen Teil der Vorderkammer (1004) des Patientenauges (10) umfassen und/oder wobei der zu behandelnde Teil (1004) des Patientenauges (10) zumindest einen Teil der Hornhaut (12) umfasst.

6. Verfahren gemäß einem der vorhergehenden Ansprüche, ferner umfassend:
- Koppeln des Patientenauges (10) an ein Kontaktinterface des Systems (100) zur laserbasierten Fehlsichtigkeitskorrektur;
wobei das Vermessen des zumindest einen Teils der vorbestimmten Strukturen des Patientenauges (10) mittels des OCT-Systems (109) zumindest einmal vor dem Koppeln des Patientenauges (10) an das Kontaktinterface und zumindest einmal nach dem Koppeln des Patientenauges (10) an das Kontaktinterface erfolgt.

7. Verfahren gemäß einem der vorhergehenden Ansprüche, ferner umfassend:
- Überprüfen der Positionierung und/oder Orientierung des zu behandelnden Teils (1004) des Patientenauges (10) relativ zum System (100) mittels eines Eye-Trackers, und optional
- Referenzieren einer mittels des Eye-Trackers bestimmten Augenposition unter Verwendung der OCT-Vermessungsdaten (1008), wobei die bestimmte Augenposition optional eine Puillenposition und/oder eine Limbusposition aufweist oder dieser entspricht.

8. Verfahren gemäß einem der vorhergehenden Ansprüche, wobei die vorgegebenen pre-operativen Vermessungsdaten (1008) pre-operative OCT-Vermessungsdaten (1008) umfassen, und wobei die Vergleichsdaten optional eine Lateralverschiebung (Δ*_{OCT}*) und/oder eine Verdrehung (Δ*β_{OCT}*) und/oder eine Verkippung (Δ*α_{OCT}*) der OCT-Vermessungsdaten (1008) relativ zu den pre-operativen OCT-Vermessungsdaten (1000) umfassen.

9. System (100) zur laserbasierten Fehlsichtigkeitskorrektur eines Patientenauges (10), das System (100) umfassend
- ein OCT-System (109) zur Vermessung von vorbestimmten Strukturen des Patientenauges (10) unmittelbar vor und/oder während einer Behandlung zur Fehlsichtigkeitskorrektur des Patientenauges (10) und zur Bereitstellung von OCT-Vermessungsdaten (1008), welche die vermessenen vorbestimmten Strukturen (1002) des Patientenauges (10) charakterisieren;
- eine Steuereinheit (106), die dazu eingerichtet ist, die von dem OCT-System (109) bereitgestellten OCT-Vermessungsdaten (1008) mit vorgegebenen pre-operativen Vermessungsdaten (1000) zu vergleichen
**dadurch gekennzeichnet, dass** das System ferner dazu eingerichtet ist, unter Verwendung von Vergleichsdaten aus dem Vergleich der vom OCT-System (109) bereitgestellten OCT-Vermessungsdaten (1008) mit vorgegebenen pre-operativen Vermessungsdaten (1000) eine Positionierung und/oder Orientierung des zu behandelnden Teils (1004) des Patientenauges (10) relativ zum System (100) zu ermitteln und das System (100) relativ zum Patientenauge (10) unter Verwendung der ermittelten Position und/oder Orientierung des zu behandelnden Teils (1004) des Patientenauges (10) auszurichten.

10. System (100) gemäß Anspruch 9, ferner umfassend eine Laserquelle (102) zur Bereitstellung einer Laserstrahlung für die Behandlung, wobei die Laserquelle (102) optional dazu ausgelegt ist, gepulste Laserstrahlung zu emittieren und wobei die Laserquelle (102) einen Excimer-Laser und/oder einen Picosekundenlaser und/oder einen Femtosekundenlaser umfasst oder als Excimer-Laser, Picosekundenlaser, Femtosekundenlaser ausgebildet ist.

11. System (100) gemäß Anspruch 10, ferner umfassend:
- eine Abbildungsoptik (124) zur Fokussierung der Laserstrahlung auf die Hornhaut (12) des Patientenauges (10), wobei die Abbildungsoptik (124) derart ausgestaltet ist, dass die Abbildungsoptik (124) ein Erfassen eines Rückreflexes (126) von Strahlung, die durch die Abbildungsoptik (124) auf das Patientenauge (10) eingestrahlt und zumindest teilweise vom Patientenauge (10) reflektiert wird, in einem Akzeptanzwinkel *χ_{Max}* von mindestens 2,5° ermöglicht.

12. System (100) gemäß Anspruch 11, wobei das System (100) und insbesondere die Abbildungsoptik (124) derart ausgebildet ist, dass der Akzeptanzwinkel *χ_{Max}* größer als 5°, optional größer als 10°, optional größer als 15°, optional größer als 25° und optional größer oder gleich 37° ist, und/oder wobei die Abbildungsoptik (124) als eine Mikroskop-Optik ausgebildet ist oder eine solche umfasst.

13. System (100) gemäß einem der Ansprüche 11 und 12, wobei die Abbildungsoptik (124) eine optische Öffnung aufweist und einen vorgegebenen Arbeitsabstand aufweist, wobei ein Durchmesser der optischen Öffnung größer als oder gleich dem vorgegebenen Arbeitsabstand ausgebildet ist, und/oder wobei die Abbildungsoptik (124) eine optische Öffnung mit einem Durchmesser von zumindest 50 mm, optional von zumindest 60 mm, aufweist und wobei die Abbildungsoptik (124) einen Arbeitsabstand aufweist, der kleiner als 50 mm und optional kleiner oder gleich 40 mm ist.

14. System (100) gemäß einem der Ansprüche 11 bis 13, wobei das Erfassen des Rückreflexes von Strahlung ein Erfassen eines Rückreflexes eines durch die Abbildungsoptik (124) in das Patientenauge (10) eingestrahlten OCT-Strahls (109a) umfasst.

15. System (100) gemäß einem der Ansprüche 9 bis 14, ferner umfassend ein Kontaktinterface zur Kopplung des Patientenauges (10) an das System (100), und/oder einen Eye-Tracker zur Überprüfung der Position und/oder Orientierung des Patientenauges relativ zum System, insbesondere relativ zur Abbildungsoptik (124).

## Claims

1. Method for aligning a system (100) for laser-based vision correction relative to a patient's eye (10) to be treated, the method comprising:
- providing specified preoperative measurement data (1000) which at least characterize predetermined structures (1002) of the patient's eye (10), the predetermined structures (1002) comprising a portion of the patient's eye (10) to be treated;
- measuring at least a portion of the predetermined structures (1002) of the patient's eye (10) by means of an OCT system (109) immediately before a treatment for vision correction for the patient's eye (10) and providing OCT measurement data (1008) which characterize the measured structures of the patient's eye;
- comparing the OCT measurement data (1008) with the specified preoperative measurement data (1000) and providing comparison data from the comparison of the OCT measurement data provided by the OCT system with the specified preoperative measurement data;
**characterized in that** the method further includes:
- determining a positioning and/or orientation of the portion of the patient's eye (10) to be treated relative to the system (100) and aligning the system (100) relative to the patient's eye (10) using the determined position and/or orientation of the portion of the patient's eye (10) to be treated.

2. Method according to Claim 1, wherein the alignment of the system (100) comprises a centration of the system (100) on the vertex of the patient's eye (10).

3. Method according to Claim 1, wherein the alignment of the system (100) comprises a centration of the system (100) on an offset position that deviates from the vertex and, optionally, a determination of offset coordinates of the offset position, and wherein the centration of the system (100) on the offset position optionally comprises a calculation of a centration-corrected fluence loss function.

4. Method according to any of the preceding claims, wherein the system (100) is aligned in manual, partially automated or fully automated fashion.

5. Method according to any of the preceding claims, wherein the predetermined structures (1002) of the patient's eye (10), which are **characterized by** the specified preoperative measurement data (1000), and the at least one portion of the predetermined structures (1002) of the patient's eye (10), which is measured by means of the OCT system (109) immediately before and/or during a treatment for vision correction, comprise at least a portion of the anterior chamber (1004) of the patient's eye (10) and/or wherein the portion (1004) of the patient's eye (10) to be treated comprises at least a portion of the cornea (12).

6. Method according to any of the preceding claims, further comprising:
- coupling the patient's eye (10) to a contact interface of the system (100) for laser-based vision correction;
wherein the at least one portion of the predetermined structures of the patient's eye (10) is measured by means of the OCT system (109) at least once before the patient's eye (10) is coupled to the contact interface and at least once after the patient's eye (10) has been coupled to the contact interface.

7. Method according to any of the preceding claims, further comprising:
- verifying the positioning and/or orientation of the portion (1004) of the patient's eye (10) to be treated relative to the system (100) by means of an eye tracker, and optionally
- referencing an eye position determined by means of the eye tracker using the OCT measurement data (1008), the determined eye position optionally including, or corresponding to, a pupil position and/or a limbus position.

8. Method according to any of the preceding claims, wherein the specified preoperative measurement data (1008) comprise preoperative OCT measurement data (1008), and wherein the comparison data optionally comprise a lateral displacement (Δ*_{OCT}*) and/or a rotation (Δ*β_{OCT}*) and/or a tilt (Δ*α_{OCT}*) of the OCT measurement data (1008) relative to the preoperative OCT measurement data (1000).

9. System (100) for laser-based vision correction for a patient's eye (10), the system (100) comprising
- an OCT system (109) for measuring predetermined structures of the patient's eye (10) immediately before and/or during a treatment for vision correction for the patient's eye (10) and for providing OCT measurement data (1008) which characterize the measured predetermined structures (1002) of the patient's eye (10);
- a control unit (106) configured to compare the OCT measurement data (1008) provided by the OCT system (109) with specified preoperative measurement data (1000),
**characterized in that** the system is further configured to determine a positioning and/or orientation of the portion (1004) of the patient's eye (10) to be treated relative to the system (100) using comparison data from the comparison between the OCT measurement data (1008) provided by the OCT system (109) and specified preoperative measurement data (1000), and to align the system (100) relative to the patient's eye (10) using the determined position and/or orientation of the portion (1004) of the patient's eye (10) to be treated.

10. System (100) according to Claim 9, further comprising a laser source (102) for providing laser radiation for the treatment, the laser source (102) optionally being designed to emit pulsed laser radiation and the laser source (102) comprising an excimer laser and/or a picosecond laser and/or a femtosecond laser or being in the form of an excimer laser, picosecond laser and/or femtosecond laser.

11. System (100) according to Claim 10, further comprising:
- an imaging optical unit (124) for focusing the laser radiation on the cornea (12) of the patient's eye (10), the imaging optical unit (124) being designed such that the imaging optical unit (124) allows a detection of a back reflection (126) of radiation radiated on the patient's eye (10) by the imaging optical unit (124) and at least partially reflected by the patient's eye (10), within an acceptance angle *χ_{Max}* of at least 2.5°.

12. System (100) according to Claim 11, wherein the system (100) and in particular the imaging optical unit (124) are designed such that the acceptance angle *χ_{Max}* is greater than 5°, optionally greater than 10°, optionally greater than 15°, optionally greater than 25° and optionally greater than or equal to 37°, and/or wherein the imaging optical unit (124) is in the form of, or comprises, a microscope optical unit.

13. System (100) according to either of Claims 11 and 12, wherein the imaging optical unit (124) has an optical opening and has a given working distance, a diameter of the optical opening being greater than or equal to the given working distance, and/or wherein the imaging optical unit (124) has an optical opening with a diameter of at least 50 mm, optionally of at least 60 mm, and wherein the imaging optical unit (124) has a working distance of less than 50 mm and optionally less than or equal to 40 mm.

14. System (100) according to any of Claims 11 to 13, wherein the detection of the back reflection of radiation comprises a detection of a back reflection of an OCT beam (109a) radiated into the patient's eye (10) by the imaging optical unit (124).

15. System (100) according to any of Claims 9 to 14, further comprising a contact interface for coupling the patient's eye (10) to the system (100), and/or an eye tracker for verifying the position and/or orientation of the patient's eye relative to the system, more particularly relative to the imaging optical unit (124).

## Revendications

1. Procédé d'orientation d'un système (100) de correction d'amétropie par laser par rapport à un œil à traiter (10) d'un patient, le procédé comprenant les étapes consistant à :
- fournir des données de mesure préopératoires prédéfinies (1000), lesquelles caractérisent au moins des structures prédéterminées (1002) de l'œil (10) du patient, les structures prédéterminées (1002) comprenant une partie à traiter de l'œil (10) du patient ;
- mesurer au moins une partie des structures prédéterminées (1002) de l'œil (10) du patient au moyen d'un système OCT (109) immédiatement avant un traitement destiné à corriger l'amétropie de l'œil (10) du patient, fournir des données de mesure OCT (1008), lesquelles caractérisent les structures mesurées de l'œil du patient ;
- comparer les données de mesure OCT (1008) et les données de mesure préopératoires prédéfinies (1000), et fournir des données de comparaison issues de la comparaison des données de mesure OCT fournies par le système OCT avec les données de mesure préopératoires prédéfinies ;
**caractérisé en ce que** le procédé comprend en outre l'étape consistant à :
- déterminer un positionnement et/ou une orientation de la partie à traiter de l'œil (10) du patient par rapport au système (100) et l'orientation du système (100) par rapport à l'œil (10) du patient à l'aide de la position et/ou de l'orientation déterminée de la partie à traiter de l'œil (10) du patient.

2. Procédé selon la revendication 1, dans lequel l'orientation du système (100) comprend le centrage du système (100) sur le vertex de l'œil (10) du patient.

3. Procédé selon la revendication 1, dans lequel l'orientation du système (100) comprend un centrage du système (100) sur une position de décalage s'écartant du vertex et, facultativement, une détermination de coordonnées de décalage de la position de décalage, et dans lequel le centrage du système (100) sur la position de décalage comprend facultativement un calcul d'une fonction de perte de fluence corrigée par centrage.

4. Procédé selon l'une quelconque des revendications précédentes, dans lequel le système (100) est orienté manuellement, de manière partiellement automatisée ou de manière entièrement automatisée.

5. Procédé selon l'une quelconque des revendications précédentes, dans lequel les structures prédéterminées (1002) de l'œil (10) du patient, qui sont **caractérisées par** les données de mesure préopératoires prédéterminées (1000), et ladite au moins une partie des structures prédéterminées (1002) de l'œil (10) du patient, qui est mesurée au moyen du système OCT (109) immédiatement avant et/ou pendant un traitement de correction d'amétropie, comprennent au moins une partie de la chambre antérieure (1004) de l'œil (10) du patient et/ou dans lequel la partie à traiter (1004) de l'œil (10) du patient comprend au moins une partie de la cornée (12).

6. Procédé selon l'une quelconque des revendications précédentes, comprenant en outre les étapes consistant à :
- coupler l'œil (10) du patient à une interface de contact du système (100) pour la correction d'amétropie par laser ;
la mesure de ladite au moins une partie des structures prédéterminées de l'œil (10) du patient au moyen du système OCT (109) étant effectuée au moins une fois avant le couplage de l'œil (10) du patient à l'interface de contact et au moins une fois après le couplage de l'œil (10) du patient à l'interface de contact.

7. Procédé selon l'une quelconque des revendications précédentes, comprenant en outre les étapes consistant à :
- vérifier le positionnement et/ou l'orientation de la partie à traiter (1004) de l'œil (10) du patient par rapport au système (100) au moyen d'un suiveur oculaire, et facultativement
- référencer une position oculaire déterminée au moyen du suiveur oculaire en utilisant les données de mesure OCT (1008), la position oculaire déterminée présentant facultativement une position de pupille et/ou une position de limbe ou correspondant à celle-ci.

8. Procédé selon l'une quelconque des revendications précédentes, dans lequel les données de mesure prédéfinies (1008) comprennent des données de mesure OCT préopératoires (1008), et dans lequel les données de comparaison comprennent facultativement un décalage latéral (Δ*_{OCT}*) et/ou une rotation (Δ*β_{OCT}*) et/ou une inclinaison (Δ*α_{OCT}*) des données de mesure OCT (1008) par rapport aux données de mesure OCT préopératoires (1000).

9. Système (100) de correction d'amétropie par laser d'un œil (10) d'un patient, le système (100) comprenant
- un système OCT (109) pour mesurer des structures prédéterminées de l'œil (10) du patient immédiatement avant et/ou pendant un traitement de correction d'amétropie de l'œil (10) du patient et pour fournir des données de mesure OCT (1008), lesquelles caractérisent les structures prédéterminées (1002) mesurées de l'œil (10) du patient ;
- une unité de commande (106), qui est conçue pour comparer les données de mesure OCT (1008) fournies par le système OCT (109) avec des données de mesure pré-opératoires prédéfinies (1000),
**caractérisé en ce que** le système est en outre conçu pour déterminer, en utilisant des données de comparaison issues de la comparaison des données de mesure OCT (1008) fournies par le système OCT (109) avec des données de mesure préopératoires prédéfinies (1000), un positionnement et/ou une orientation de la partie (1004) à traiter de l'œil (10) du patient par rapport au système (100), et pour orienter le système (100) par rapport à l'œil (10) du patient en utilisant la position et/ou l'orientation déterminée de la partie (1004) à traiter de l'œil (10) du patient.

10. Système (100) selon la revendication 9, comprenant en outre une source laser (102) pour fournir un rayonnement laser destiné au traitement, la source laser (102) étant facultativement conçue pour émettre un rayonnement laser pulsé et la source laser (102) comprenant un laser excimère et/ou un laser picoseconde et/ou un laser femtoseconde ou étant conçue sous forme de laser excimère, de laser picoseconde, de laser femtoseconde.

11. Système (100) selon la revendication 10, comprenant en outre :
- une optique d'imagerie (124) pour focaliser le rayonnement laser sur la cornée (12) de l'œil (10) du patient, l'optique d'imagerie (124) étant conçue de telle sorte que l'optique d'imagerie (124) permette une détection d'une rétroréflexion (126) du rayonnement qui est rayonné vers l'œil (10) du patient par l'optique d'imagerie (124) et qui est au moins partiellement réfléchi par l'œil (10) du patient sous un angle d'acceptation *χ_{Max}* d'au moins 2,5°.

12. Système (100) selon la revendication 11, le système (100) et en particulier l'optique d'imagerie (124) étant réalisés de telle sorte que l'angle d'acceptation *χ_{Max}* soit supérieur à 5°, facultativement supérieur à 10°, facultativement supérieur à 15°, facultativement supérieur à 25° et facultativement supérieur ou égal à 37°, et/ou l'optique d'imagerie (124) étant conçue sous la forme d'une optique de microscope ou comprenant une optique de microscope.

13. Système (100) selon l'une quelconque des revendications 11 et 12, dans lequel l'optique d'imagerie (124) présente une ouverture optique et une distance de travail prédéfinie, un diamètre de l'ouverture optique étant conçu pour être supérieur ou égal à la distance de travail prédéfinie, et/ou l'optique d'imagerie (124) présentant une ouverture optique ayant un diamètre d'au moins 50 mm, facultativement d'au moins 60 mm, et l'optique d'imagerie (124) présentant une distance de travail qui est inférieure à 50 mm et facultativement inférieure ou égale à 40 mm.

14. Système (100) selon l'une quelconque des revendications 11 à 13, dans lequel la capture de la rétroréflexion du rayonnement comprend la détection d'une rétroréflexion d'un faisceau OCT (109a) injecté dans l'œil (10) du patient par l'optique d'imagerie (124).

15. Système (100) selon l'une quelconque des revendications 9 à 14, comprenant en outre une interface de contact destinée à coupler l'œil (10) du patient au système (100), et/ou un suiveur oculaire pour vérifier la position et/ou l'orientation de l'œil du patient par rapport au système, en particulier par rapport à l'optique d'imagerie (124).
